## Europäisches Patentamt
## European Patent Office
## Office européen des brevets

(19)

(11) Veröffentlichungsnummer: **0 131 178**
**B1**

(12)

# EUROPÄISCHE PATENTSCHRIFT

(45) Veröffentlichungstag der Patentschrift:
**02.11.88**

(21) Anmeldenummer: **84106965.1**

(22) Anmeldetag: **18.06.84**

(51) Int. Cl.⁴: **A 61 F 2/30**

(54) Hüftgelenk-Endoprothese mit einem im Oberschenkelknochen zu verankernden Schaft.

(30) Priorität: **27.06.83 DE 3323131**

(43) Veröffentlichungstag der Anmeldung:
**16.01.85 Patentblatt 85/3**

(45) Bekanntmachung des Hinweises auf die Patenterteilung:
**02.11.88 Patentblatt 88/44**

(84) Benannte Vertragsstaaten:
**AT BE CH DE FR GB IT LI LU NL SE**

(56) Entgegenhaltungen:
**EP-A-0 085 147**
**EP-A-0 099 642**
**WO-A-84/03037**
**DE-A-2 839 092**
**FR-A-1 278 359**
**FR-A-2 419 717**
**GB-A-817 525**

(73) Patentinhaber: **Waldemar Link GmbH & Co,**
**Barkhausenweg 10, D-2000 Hamburg 63 (DE)**

(72) Erfinder: **Link, Helmut D., Wildstieg 14, D-2000**
**Hamburg 65 (DE)**

(74) Vertreter: **Glawe, Delfs, Moll & Partner**
**Patentanwälte, Postfach 26 01 62 Liebherrstrasse**
**20, D-8000 München 26 (DE)**

EP 0 131 178 B1

LIBER, STOCKHOLM 1988

## Beschreibung

Die Erfindung betrifft eine Hüftgelenkendoprothese mit einem im Oberschenkelknochen zu verankernden, sich von oben nach unten verjüngenden Schaft.

Bei einer zum nicht vorveröffentlichten Stand der Technik gehörenden Prothese (EP-A-85 147) setzt sich der Schaft aus einem mit dem Gelenkkopf verbundenen Hauptteil und einem über zusammenwirkende Führungsflächen daran in Längsrichtung beweglich geführten Keilstück zusammen, das eine gegenüber dem Knochengewebe haftungsfördernd ausgebildete Außenfläche aufweist. Das Keilstück ist auf der lateralen Seite des Hauptteils des Schafts vorgesehen. Um eine funktionsgerechte Lage des Gelenkkopfes der Prothese sicherzustellen, wird zunächst der den Gelenkkopf tragende Hauptteil des Schafts in den Knochen eingeschlagen. Anschließend wird das Keilstück bei festgehaltenem Hauptteil des Schafts eingeschlagen, wobei die Keilform des Keilstücks ein Verklemmen des Schafts im Knochen bewirkt, ohne daß die Höhenlage des Hauptteils des Schafts im Knochen verändert wird. Wenn sich der Schaft lockert, beispielsweise aufgrund unphysiologischer Belastung oder von Alterungserscheinungen, kann dies dadurch korrigiert werden, daß in einem späteren Zeitpunkt im Rahmen einer Nachoperation das Keilstück weiter eingeschlagen wird. In jedem Falle ist bei dieser Prothese vorgesehen, daß der Hauptteil des Schafts eine vorbestimmte Höhe gegenüber dem Knochen inne hat und beibehält und lediglich die Keilwirkung des Keilstücks durch Einschlagen zum Verklemmen der Prothese im Knochenrohr genutzt wird. Die Möglichkeit der Lockerung der Prothese durch Erweiterung des den Schaft aufnehmenden Knochens ist bei der bekannten Prothese nicht ausgeschlossen, und es ist jedenfalls eine Nachoperation erforderlich, um die Lockerung durch Nachschlagen des Keilstücks zu beseitigen.

Bei einer gleichfalls zum nicht vorveröffentlichten Stand der Technik gehörenden Prothese (WO-A-84/03037) sind an der Vorder-und Rückseite des Prothesenschafts Führungsnuten zur Aufnahme von Keilen vorgesehen, die als Sicherheit gegen Rotation des Schafts im Knochen und zum Ausgleich des Spiels zwischen Prothesenschaft und Knochen eingeschlagen werden. Die Reibungsverhältnisse zwischen den Keilen und dem Schaft sowie dem Knochen werden ebensowenig bedacht wie die Möglichkeit einer späteren Lockerung.

Vorbekannt ist eine Prothese (FR-A-1 278 359), deren Schaft lateral eine Nut zur Aufnahme einer Rippe aufweist, die nach dem Einsetzen des Schafts eingeschlagen wird, um eine Rotationssicherung und eine Abstützung des Schafts an der lateralen Kortikalis zu bilden. Zu diesem Zweck wird sie nach dem Einsetzen durch Schrauben fest mit dem Schaft verbunden.

Der Erfindung liegt die Aufgabe zugrunde, eine Endoprothese zu schaffen, die auch bei Erweiterung des den Prothesenschaft aufnehmenden Markkanals nicht oder weniger zur Lockerung neigt. Die erfindungsgemäße Lösung besteht darin, daß der sich von oben nach unten verjüngende Schaft aus einem mit dem Gelenkkopf verbundenen Hauptteil und einer Mehrzahl von über zusammenwirkende Führungsflächen daran in Längsrichtung beweglich geführten Keilstücken zusammensetzt, die an voneinander abgewandten Seiten des Schafts vorgesehen sind und eine gegenüber dem Knochengewebe haftungsfördernd ausgebildete Außenfläche aufweisen, wobei die keilförmig zueinander verlaufenden Führungsflächen zur Erzeugung einer geringeren Haftkraft als die Außenflächen der Keilstücke ausgebildet sind.

Von dem an erster Stelle oben angegebenen Stand der Technik unterscheidet sich die erfindungsgemäße Prothese grundsätzlich dadurch, daß die Keilstücke in der Regel im Knochen eine durch die Operation einmalig festgelegte Höhe aufweisen, während der Hauptteil des Schafts, der den Gelenkkopf trägt, in Längsrichtung verschiebbar ist, und zwar selbsttätig aufgrund der natürlichen Belastung, die danach strebt, den Hauptteil des Schafts zwischen den Keilstücken weiter in den Knochen einzuschieben. Eine Erweiterung des Knochenrohrs wird auf diese Weise selbsttätig durch eine Spreizung der Keilstücke unter entsprechender Senkung des Hauptteils des Prothesenschafts ausgeglichen. Zwar ist dies auch mit einer Senkung des Gelenkkopfs verbunden; die physiologischen Gegebenheiten werden dadurch aber nur geringfügig geändert, wobei durch entsprechende Wahl des Keilwinkels, den der Hauptteil des Schafts zwischen den Führungsflächen der Keilstücke bildet, dafür gesorgt werden kann, daß selbst bei größter denkbarer Markkanalerweiterung die Absenkungsstrecke des Gelenkkopfs innerhalb der Toleranzgrenzen bleibt.

Die selbstnachstellende Wirkung der erfindungsgemäßen Prothese wird dadurch ermöglicht, daß die Haftung der Außenflächen der Keilstücke am Knochengewebe größer ist als die Haftung, die in den zusammenwirkenden Führungsflächen des Hauptteils des Schafts und der Keilstücke wirkt. Dem Fachmann sind viele Möglichkeiten bekannt, die Oberfläche eines Prothesenschafts gegenüber dem Knochengewebe haftungsfördernd auszubilden, beispielsweise durch Erhöhungen und Vertiefungen, Oberflächenporosität, Durchbrechungen, in die das Knochengewebe einwächst.

Ferner sind ihm Möglichkeiten bekannt, die zwischen den Führungsflächen wirkende Haftkraft vorherzubestimmen, beispielsweise durch Oberflächenrauheit oder im Querschnitt keilförmige Gestaltung der Flächen.

Die Wirkung der Erfindung ist gebunden an die keilförmige Ausbildung des Hauptteils des Schafts zwischen den Führungsflächen. Vorteilhaft, aber nicht unbedingt erforderlich dafür, ist eine keilförmige Ausbildung der Keilstücke. Vielmehr ist es denkbar, daß die an den Keilstücken vorgesehenen Führungsflächen parallel zu deren Außenflächen im Längsschnitt verlaufen oder daß sich die Keilstücke sogar von distal nach proximal verjüngen, so daß der an den Führungsflächen wirkende Keilwinkel größer ist als der natürliche Keilwinkel im sich nach proximal erweiternden Markkanal des Oberschenkelknochens.

Es ist zwar möglich, die mit dem Knochengewebe zusammenwirkende Umfangsfläche des Prothesenschafts im oberen Abschnitt desselben ausschließlich von den Keilstücken bilden zu lassen. Vorteilhafter ist es aber im allgemeinen, wenn außer den Keilstücken auch der Hauptteil des Prothesenschafts an der Bildung der Umfangsfläche beteiligt ist. Dadurch wird sichergestellt, daß normalerweise, solange eine Erweiterung des Markkanals nicht stattfindet, die Verankerungskräfte gänzlich oder zu einem wesentlichen Teil von der Oberfläche des Hauptteils des Schafts auf den Knochen übertragen werden, so daß eine Verkeilungsbewegung erst dann stattfinden kann, wenn die Oberfläche des Hauptteils des Prothesenschafts in Folge von Erweiterung des Knochenrohrs den Kontakt mit dem Knochengewebe verliert und dadurch stärker in die Markhöhle einsinkt, bis die Anordnung durch Spreizung der Keilstücke wieder Halt gefunden hat. Eine besonders vorteilhafte Ausführung besteht beispielsweise darin, daß vom Hauptteil des Schafts eine Mehrzahl schaftfester Rippen zur Umfangsfläche vorragt und in den Rippenzwischenräumen die Keilstücke angeordnet sind.

Damit die erwähnte Kraftübertragung von dem Hauptteil des Schafts auf das Knochengewebe stattfinden kann, hat auch dieser zweckmäßigerweise eine in bezug auf das Knochengewebe haftungsfördernd ausgebildete Oberflächenstruktur, wobei sogar nach einem weiteren Merkmal der Erfindung die mit dem Knochengewebe zusammenwirkende Oberfläche des Hauptteils des Schafts zur alleinigen Übertragung der Belastung hinreichend bemessen sein kann. Dadurch soll bewirkt werden, daß nicht jede kräftige Belastung der Prothese zu einer Relativbewegung des Prothesenschafts gegenüber den Keilstücken und dem Knochengewebe führt. Angestrebt wird vielmehr, daß diese Korrekturbewegung erst dann stattfindet, wenn der Verbund zwischen dem Hauptteil des Prothesenschafts und dem Knochengewebe nachläßt. Dieser Verbund kann dabei dazu beitragen, daß die Korrekturverschiebung nicht traumatisch als plötzliche Brucherscheinung unter Spitzenbelastung stattfindet, sondern als kontinuierlicher Umlagerungsprozeß, in welchem ein Gleichgewicht herrscht zwischen den über die Keilstücke und den direkt vom Hauptteil auf das Knochengewebe übertragenen Kräften.

Nach einem weiteren Merkmal der Erfindung kann vorgesehen sein, daß der Keilwinkel kleiner als der Reibungswinkel ist, damit zwar einerseits durch die Belastung der Prothese die gewünschte Spreizung der Keilstücke zwecks Ausgleichs einer Markkanalerweiterung stattfinden kann, daß aber andererseits die elastische Rückfederungsneigung des Knochengewebes nicht zu der entgegengesetzten Relativbewegung führen kann, was sonst ein ständiges "Pumpen" der Anordnung zur Folge habe könnte. Ein Mittel zur Verhinderung einer solchen ständigen Wechselbewegung besteht darin, daß die zusammenwirkenden Führungsflächen reibungsintensiv ausgeführt sind. Dann können nur solche Kräfte, die die Reibungskraft übersteigen, eine Relativbewegung hervorrufen.

Besonders vorteilhaft ist die Ausführung der reibungsintensiv geformten Führungsflächen in Gestalt einer Stufung oder Zahnung, die so ausgeführt ist, daß nur beim Überschreiten einer bestimmten Kraftschwelle ein Überspringen der Teile aus einer Rastposition in die nächste möglich ist. Bei einer Stufung in Form einer Vielzahl von Einzelstufen, setzen diese sich jeweils aus einer Fläche großen Keilwinkels (Winkel zwischen dieser Fläche und der Schaftlängsrichtung) und einer weiteren Fläche zusammen, die einen Winkel mit der Schaftlängsrichtung einschließt, der sehr klein, null oder negativ (ein sich nach unten öffnender Winkel) ist. Die in Schaftlängsrichtung wirkenden Belastungskräfte werden dabei über die Stufenflächen mit großem Keilwinkel übertragen, wobei gleichzeitig eine Spreizkraft auf die Keilstücke ausgeübt wird, die gleich dem Quotienten der Belastungskräfte durch den Tangens des Keilwinkels ist. Eine Relativbewegung findet dabei nur dann statt, wenn die Spreizkräfte größer werden als die der Spreizung entgegenwirkenden Kräfte des Knochengewebes bei einer Dehnung um die radiale Stufenhöhe. Durch Wahl eines großen Keilwinkels kann man die vom Knochengewebe auf zunehmende Spreizkraft begrenzen. Durch geeignete Bemessung der radialen Stufenhöhe (gemessen quer zur Schaftrichtung) bestimmt man, bei welchem Grad von Markkanalerweiterung jeweils ein Korrektursprung der Protheseteile stattfinden soll. Keilwinkel und Stufenhöhe werden zweckmäßigerweise so bemessen, daß die Stufenhöhe nicht oder nicht wesentlich größer ist als die bei maximaler Belastung unter Zugrundelegung des Keilwinkels zu erwartende Dehnung des Knochengewebes.

Die Erfindung setzt im allgemeinen den zementfreien oder zementarmen Einsatz des Prothesenschafts voraus.

Die Erfindung wird im folgenden näher unter Bezugnahme auf die Zeichnung erläutert, die ein vorteilhaftes Ausführungsbeispiel veranschaulicht. Darin zeigen:

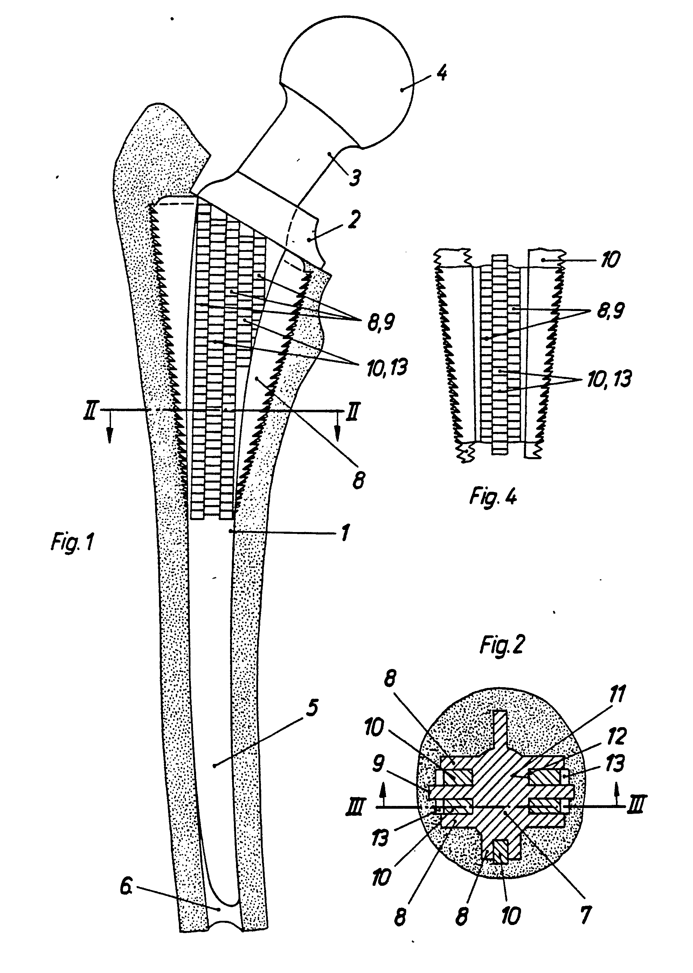

Fig. 1 eine Seitenansicht einer Hüftgelenk-Femurschaftprothese,

Fig. 2 einen Querschnitt durch den Schaft gemäß Linie II-II der Fig. 1,

Fig. 3 einen Längsschnitt größeren Maßstabs gemäß Linie IIII-III der Fig. 2, und zwar mit unterschiedlicher Ausführung der Führungsflächen auf der linken bzw. rechten Seite der Mittellinie,

Fig. 4 eine Teil-Seitenansicht in zu Fig. 1 lotrechter Projektionsrichtung und

Fig. 5 eine gestufte Ausführung der Führungsflächen.

Die Prothese besteht aus dem Schaft 1, dem gegebenenfalls auswechselbaren Kragen 2, dem Kopfhals 3 und dem Gelenkkopf 4. In seinem unteren Teil 5 ist der Schaft 1 herkömmlich derart ausgestaltet, daß er mit seiner Oberfläche im wesentlichen an der harten Kortikalis des Oberschenkelknochens anliegen kann. Im oberen Bereich, in welchem sich der Markkanal 6 zum Trochanterbereich hin stark erweitert, gehen von dem Hauptteil 7 in Schaftlängsrichtung verlaufende Rippen 8 aus, die fest mit dem Hauptteil 7 verbunden sind. Auf der Vorder- und Rückseite des Schafts sind jeweils drei solcher Rippen 8 in im Querschnitt paralleler Anordnung vorgesehen. An der Innenseite sind zwei Rippen und an der Außenseite eine Rippe angeordnet. Die Rücken der Rippen 8, d.h. die nach außen gewendeten, sind mit Zähnen 9 versehen, um sich besser im Knochengewebe verankern zu können. An der Stelle der Zähne können auch andere Oberflächengestaltungen treten, die einen guten Verbund mit dem Knochengewebe gewährleisten.

Zwischen jeweils zwei mit dem Hauptteil 7 verbundenen Rippen 8 befindet sich ein Zwischenraum konstanter Weite, der eine Führung bildet für jeweils ein Keilstück 10, das zwischen den schaftfesten Rippen in Schaftlängsrichtung beweglich ist und sich an dem die Führungsflächen bildenen Grund 11 des Rippenzwischenraums mit seiner Führungsfläche 12 abstützt. An seiner Rückenfläche trägt er Zähne 13 zur besseren Verankerung im Knochengewebe bzw. eine alternative Oberflächengestaltung. Im dargestellten Beispiel ist die Zahnung 9 der mit dem Hauptteil 7 fest verbundenen Rippen sägezahnförmig so ausgeführt, daß sie einer senkenden Relativbewegung weniger Widerstand entgegensetzt als die Zahnung 13 der beweglichen Schaftteile. Jedoch ist dies nicht unbedingt erforderlich.

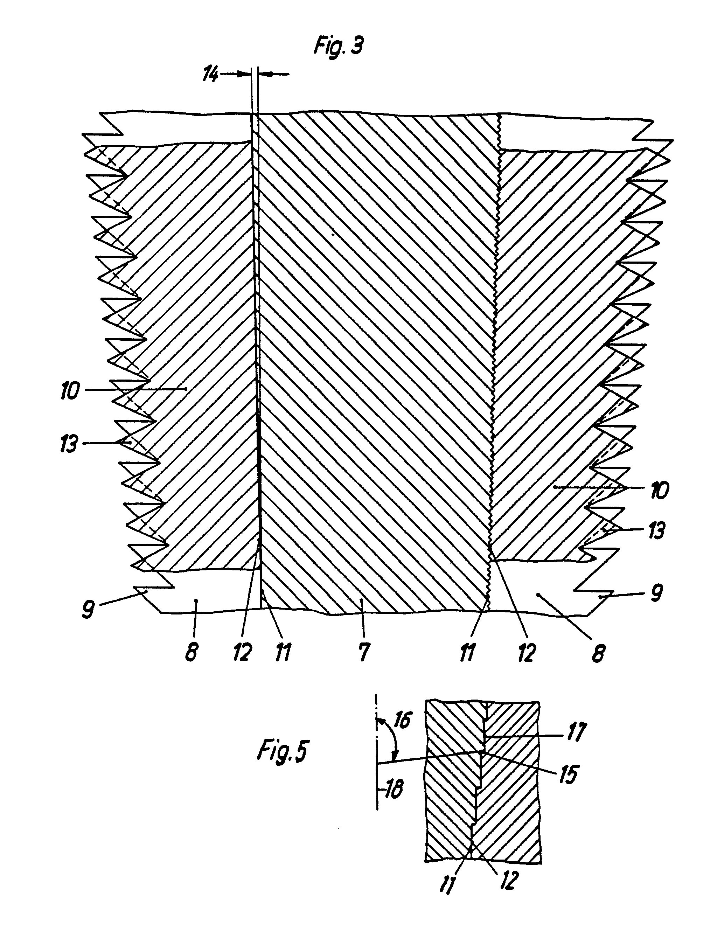

Der Abstand zwischen auf verschiedenen Seiten des Hauptteils einander gegenüberliegenden Führungsflächen 11 wird von unten nach oben größer. Das bedeutet, daß sie Keilflächen bilden, durch welche die Keilstücke 10 nach außen gedrängt werden, wenn der Schaft sich tiefer in die Markhöhle einsenkt, und dadurch zu stärkerer Haftung am Knochengewebe gezwungen werden und daher diese Einsenkbewegung nicht teilen. In dem Längsschnitt Fig. 3 erkennt man, daß der Keilwinkel 14, den die Führungsflächen 11 mit der Schaftlängsrichtung einschließen, sehr klein ist. Das bedeutet, daß die Keilstücke 10 sich an der Übertragung der Längskräfte vom Schaft auf den Knochen praktisch nicht beteiligen. Jedoch beteiligen sie sich voll an der Übertragung von quer zur Schaftlängsrichtung verlaufenden Kräften und sind daher dazu geeignet, die Sicherung des Schafts in der Markhöhle auch dann zu gewährleisten, wenn diese sich erweitert. Es ist nicht zu befürchten, daß eine ständige Hin-und Herbewegung der beweglichen und der festen Schaftteile in Längsrichtung zueinander stattfindet, wenn die Prothese wechselnden Belastungen ausgesetzt ist. Das liegt daran, daß der äußerst geringe Keilwinkel jedenfalls geringer ist als der Reibungswinkel, so daß wechselnde Querkräfte keine Längsverschiebung der Keilstücke veranlassen können.

Fig. 3 zeigt in der linken Hälfte die glatte Ausführung des Führungsflächenpaars 11, 12 und auf der rechten Seite eine gezahnte Ausführung, durch die verhindert werden soll, daß wechselnde Längskräfte zu wechselnden Verschiebungen zwischen den festen und beweglichen Prothesenteilen führen können, sofern sie unterhalb einer gewissen Schwelle bleiben.

Fig. 5 zeigt eine stufige Ausführung der Führungsflächen 11 und 12, wobei sich jede Stufe zusammensetzt aus einer Fläche mit großem Keilwinkel 16 und einer anderen Fläche 17, die etwa parallel zur Schaftlängsrichtung 18 verläuft. Es ist möglich, die verschiedenen Führungsflächenpaare an einer Prothese unterschiedlich zu gestalten. Bevorzugt wird jedoch eine gleiche Gestaltung aller Führungsflächen.

Auf der Außenseite (lateral) der dargestellten Prothese ist kein bewegliches Keilstück sondern eine fest mit dem Hauptteil des Schafts verbundene Rippe vorgesehen, weil dort weniger Druckkräfte als Zugkräfte zu übertragen sind.

## Patentansprüche

1. Hüftgelenk-Endoprothese mit einem im Oberschenkelknochen zu verankernden, sich von oben nach unten verjüngenden Schaft (1), der sich aus einem mit dem Gelenkkopf (4) verbundenen Hauptteil (7) und einer Mehrzahl von über zusammenwirkende Führungsflächen (11, 12) daran in Längsrichtung beweglich geführten Keilstücken (10) zusammensetzt, die an voneinander abgewandten Seiten des Schafts angeordnet sind und eine gegenüber dem Knochengewebe haftungsfördernd ausgebildete Außenfläche (13) aufweisen, wobei die keilförmig zueinander verlaufenden Führungsflächen (11, 12) zur Erzeugung einer geringeren Haftkraft als die Außenflächen (13) der Keilstücke (10) ausgebildet sind.

2. Hüftgelenk-Endoprothese nach Anspruch 1, dadurch gekennzeichnet, daß außer den Keilstücken (10) auch der Hauptteil (7) des Schafts (1) an der Bildung der Außenfläche des Schafts beteiligt ist.

3. Hüftgelenk-Endoprothese nach Anspruch 1 oder 2, dadurch gekennzeichnet, daß der Hauptteil (7) des Schafts (1) dem Knochengewebe zugewendete Außenflächen (9) mit haftungsfördernd ausgebildeter Oberflächenstruktur aufweist.

4. Hüftgelenk-Endoprothese nach einem der Ansprüche 1 bis 3, dadurch gekennzeichnet, daß die von dem Hauptteil (7) des Schafts (1) gebildete Außenfläche zur alleinigen Übertragung der Prothesenbelastung hinreichend bemessen ist.

5. Hüftgelenk-Endoprothese nach einem der Ansprüche 1 bis 4, dadurch gekennzeichnete, daß eine Mehrzahl fest mit dem Hauptteil (7) des Schafts (1) verbundener, mit ihrer Rückenfläche (9) an der Bildung der Schaftaußenfläche beteiligter Rippen (8) nach außen vorragt und daß in den Rippenzwischenräumen die Keilstücke (10) angeordnet sind.

6. Hüftgelenk-Endoprothese nach einem der Ansprüche 1 bis 5, dadurch gekennzeichnet, daß der Keilwinkel (14) der zusammenwirkenden Führungsflächen (11, 12) kleiner ist als der Reibungswinkel.

7. Hüftgelenk-Endoprothese nach einem der Ansprüche 1 bis 6, dadurch gekennzeichnet, daß die zusammenwirkenden Führungsflächen (11, 12) reibungsintensiv ausgeführt sind.

8. Hüftgelenk-Endoprothese nach Anspruch 7, dadurch gekennzeichnet, daß die Führungsflächen (11, 12) gezahnt ausgeführt sind.

9. Hüftgelenk-Endoprothese nach Anspruch 7, dadurch gekennzeichnet, daß die Führungsflächen (11, 12) gestuft ausgeführt sind.

10. Hüftgelenk-Endoprothese nach Anspruch 9, dadurch gekennzeichnet, daß eine vielzahl von Keilflächenstufen vorgesehen ist, die sich jeweils aus einer Fläche (15) großen Keilwinkels (16) und einer Fläche (17) geringen Keilwinkels zusammensetzen.

## Claims

1. A hip joint endoprosthesis with a downwardly tapering shaft (1) to be anchored in the femur, which shaft is composed of a major portion (7) connected to the joint head (4) and of a plurality of wedge pieces (10) which via cooperating guide surfaces (11, 12) are guided movably thereon in longitudinal direction and which are disposed on mutually remote sides of the shaft and have an outer surface (13) formed to promote adhesion with respect to the bone tissue, wherein the guide surfaces (11, 12) extending in wedge-shaped manner relative to one another are formed to provide less adhesion than the outer surfaces (13) of the wedge pieces (10).

2. A hip joint endoprosthesis according to Claim 1, characterised in that in addition to the wedge pieces (10) the major portion (7) of the shaft (1) also assists in forming the outer surface of the shaft.

3. A hip joint endoprosthesis according to Claim 1 or 2, characterised in that the major portion (7) of the shaft (1) has outer surfaces (9) of a surface structure formed with adhesion-promoting properties and facing the bone tissue.

4. A hip joint endoprosthesis according to any one of Claims 1 to 3, characterised in that the outer surface formed by the major portion (7) of the shaft (1) is of sufficient dimensions to serve for the sole transmission of the prosthesis load.

5. A hip joint endoprosthesis according to any one of Claims 1 to 4, characterised in that a plurality of ribs (8) protrudes outwardly, which ribs are securely connected to the major portion (7) of the shaft (1) and which with their back surface (9) assist in forming the shaft outer surface, and in that the wedge pieces (10) are disposed in the rib interspaces.

6. A hip joint endoprosthesis according to any one of Claims 1 to 5, characterised in that the wedge angle (14) of the cooperating guide surfaces (11, 12) is smaller than the angle of friction.

7. A hip joint endoprosthesis according to any one of Claims 1 to 6, characterised in that the cooperating guide surfaces (11, 12) are of frictionally intensive form.

8. A hip joint endoprosthesis according to Claim 7, characterised in that the guide surfaces (11, 12) are of toothed form.

9. A hip joint endoprosthesis according to Claim 7, characterised in that the guide surfaces (11, 12) are of stepped form.

10. A hip joint endoprosthesis according to Claim 9, characterised in that a plurality of wedge surface steps are provided, each of which is composed of a surface (15) of large wedge angle (16) and of a surface (17) of small wedge angle.

## Revendications

1. Endoprothèse de hanche, comportant une tige (1) destinée à l'ancrage dans le fémur, tige qui s'amincit de haut en bas et qui se compose d'une partie principale (7) raccordée à la tête articulaire (4) et de plusieurs pièces de coinçage (10) guidées au moyen de surfaces de guidage associées (11, 12) de manière à pouvoir être déplacées en direction longitudinale sur ces surfaces, pièces de coinçage qui sont disposées sur des côtés mutuellement opposés de la tige et qui présentent une surface extérieure (13) favorisant l'adhérence au tissu osseux, les surfaces de guidage (11, 12) qui concourent en forme de coin étant réalisées de manière à produire une force d'adhérence plus faible que les surfaces extérieures (13) des pièces de coinçage (10).

2. Endoprothèse de hanche selon la revendication 1, caractérisée en ce qu'en dehors des pièces de coinçage (10), la partie principale (7) de la tige (1) participe également à la formation de la surface extérieure de la tige.

3. Endoprothèse de hanche selon la revendication 1 ou 2, caractérisée en ce que la partie principale (7) de la tige (1) présente des surfaces extérieures (9) dirigées vers le tissu osseux qui ont une structure superficielle réalisée de façon à favoriser l'adhérence.

4. Endoprothèse de hanche selon l'une quelconque des revendications 1 à 3, caractérisée en ce que la surface extérieure formée par la partie principale (7) de la tige (1) est dimensionnée assez largement pour la transmission exclusive de la charge à laquelle est soumise la prothèse.

5. Endoprothèse de hanche selon l'une quelconque des revendications 1 à 4, caractérisée en ce que plusieurs nervures (8), solidaires de la partie principale (7) de la tige (1) et participant par leur face arrière (9) à la formation de la surface extérieure de la tige, font saillie vers l'extérieur et en ce que les pièces de coinçage (10) sont disposées dans les intervalles entre ces nervures.

6. Endoprothèse de hanche selon l'une quelconque des revendications 1 à 5, caractérisée en ce que l'angle de coincement (14) des surfaces de guidage associées (11, 12) est plus petit que l'angle de frottement.

7. Endoprothèse de hanche selon l'une quelconque des revendications 1 à 6, caractérisée en ce que les surfaces de guidage associées (11, 12) sont réalisées de façon à produire un frottement intense.

8. Endoprothèse de hanche selon la revendication 7, caractérisée en ce que les surfaces de guidage (11, 12) sont réalisées sous forme striée.

9. Endoprothèse de hanche selon la revendication 7, caractérisée en ce que les surfaces de guidage (11, 12) sont réalisées sous forme de gradins.

10. Endoprothèse de hanche selon la revendication 9, caractérisée en ce qu' il est prévu une multiplicité de gradins de surfaces de coincement, se composant chacun d'une surface (15) à grand angle de coincement (16) et d'une surface (17) dont l'angle de coincement est plus petit.

Fig. 1

Fig. 2

Fig. 4

**Fig. 3**

**Fig. 5**